# EUROPEAN PATENT APPLICATION

(11) **EP 2 042 158 A1**
(43) Date of publication of application: **01.04.2009**
(21) Application number: 07291078.9
(22) Date of filing: 07.09.2007
(51) Int. Cl.: A61K 8/81, A61K 8/35, A61Q 17/04

(54) **Use of alpha olefin copolymers as photostabilizing agents in sunscreen compositions**

(71) Applicant: Johnson & Johnson Consumer France SAS, 92787 Issy les Moulineaux Cedex 9 (FR)
(72) Inventor: Saclier, Sebastien, 27400 Montaure (FR); Tischenbach, Isabelle, 27400 Louviers (FR); Moulin, Sabrina, 27150 Puchay (FR)
(74) Representative: Metten, Karl-Heinz

(57) **Abstract**

The invention provides the use of an alpha olefin copolymer as a photostabilizing agent in a sunscreen composition comprising a dibenzoylmethane derivative UV-A absorbing agent, such as avobenzone. When alpha olefin copolymers are used said compositions preferably have a photostability decrease of less than about 1/3 of the photostability decrease of a similar composition that is the same as the sunscreen composition but not containing said alpha olefin copolymer.

## Description

The present invention relates to the use of alpha olefin copolymers as photostabilizing agents in sunscreen compositions comprising a dibenzoylmethane derivative UV-A absorbing agent such as avobenzone. When alpha olefin copolymers are used said compositions preferably have a photostability decrease of less than about 1/3 of the photostability decrease of a composition that is the same as the sunscreen composition but not containing said alpha olefin copolymer.

### BACKGROUND OF THE INVENTION

The prolonged exposure to ultra-violet (UV) radiation, such as from the sun, can lead to the formation of light dermatoses and erythemas, as well as increase the risk of skin cancers, such as melanoma. UV radiation can also accelerate skin aging, such as loss of skin elasticity and wrinkling. Radiation of wavelengths in the UV-A range (from about 320 to 400 nm) and the UV-B range (from about 280 to about 320 nm) can cause such skin damage, and, thus, sunscreen products should preferably comprise both UV-A and UV-B sun filters.

Numerous UV-B absorbers are available for sunscreening purposes, however there are far fewer choices available for UV-A protection, particularly in the longer wavelength regions of the UV-A (340-400nm). Also hampering the efforts to provide high UV-A protection are strict regulations on the concentration of UV-A filters that can be included in sunscreen products.

Avobenzone (4-(1,1-dimethylethyl)-4'-methoxydibenzoylmethane, sold for example as PARSOL 1789 by Roche Vitamins and Fine Chemicals) is a commonly used UV-A filter. However, it is known to lack photostability. Much effort has been made to provide avobenzone-containing sunscreen compositions that are photostable. For instance, US Patent No. 5,993,789 discloses the use of diethylhexyl naphthalates as photostabilizers in such compositions. US Patent No. 6,485,713 discloses the use of amide, bis-urethane and malate solvents to control the rate of photodecay of UV filters. US Patent No. 7,166,273 discloses the use of compounds of the following formula as photostabilizers in avobenzone-containing sunscreen compositions: 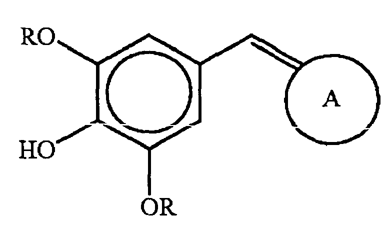 wherein A is a moiety that provides chromophoric properties within the UV radiation range of 290-400 nm. This moiety comprises one divalent group or two monovalent groups with at least one group having carbonyl (C=O) functionality. For the above formula, each R is independently linear or branched C1-C8 alkyl.

US Patent No. 7,166,273 additionally discloses the presence of PERFORMA V1608, a C30-38 alpha olefin/isopropyl maleate/maleic anhydride copolymer commercially available from New Phase Technologies, in the formulations therein. However, this ingredient is not taught or suggested to affect photostability, which is instead controlled using compounds of the above formula. See also US Patent Nos. 6,395,269 and 6,899,866 and US Patent Application Publication No. 2004/0047819.

The present invention relates to the use of at least one alpha olefin copolymer as a photostabilizing agent in sunscreen compositions comprising at least one dibenzoylmethane derivative UV-A absorbing agent, in particular avobenzone. When alpha olefin copolymers are used as photostabilizers in said sunscreen compositions there is no need for additionally using known photostabilizers. When using compositions said alpha olefin copolymers as photostabilizing agent in sunscreen compositions said compositions usually have a photostability decrease of less than about 1/3 of the photostability decrease of a similar composition that is the same as the sunscreen composition but not containing said alpha olefin copolymer.

The present invention relates to a sunscreen composition comprising at least one dibenzoylmethane derivative UV-A absorbing agent, in particular avobenzone, which has surprising photostability without the need for known photostabilizers. The composition further comprises at least one alpha olefin copolymer. It preferablyhas a photostability decrease of less than about 1/3 the photostability decrease of a similar composition that is the same as the sunscreen composition but not containing said alpha olefin copolymer.

### SUMMARY OF THE INVENTION

The invention relates to the use of at least one alpha olefin copolymer as a photostabilizing agent in sunscreen compositions comprising:
(a) at least one dibenzoylmethane derivative UV-A absorbing agent, in particular of the formula: 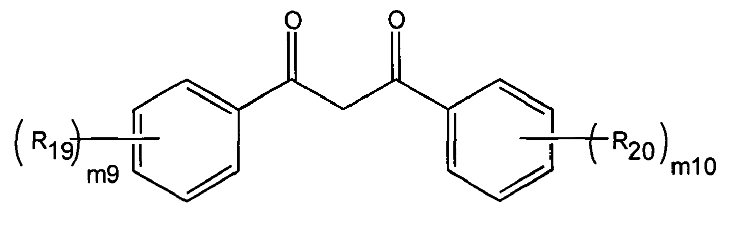 wherein R₁₉ and R₂₀, independently, are C1-C8 alkyl or C1-C8 alkoxy, m⁹ is 0 to 3, and m¹⁰ is 1 to 3. Said compositions preferably have a photostability decrease of less than about 1/3 the photostability decrease of a similar composition that is the same as the sunscreen composition but not containing said alpha olefin copolymer.

The invention relates to a sunscreen composition comprising:
(a) at least one dibenzoylmethane derivative UV-A absorbing agent, in particular of the formula:

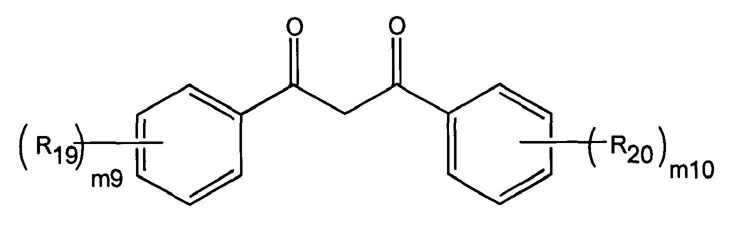

wherein R₁₉ and R₂₀, independently, are C1-C8 alkyl or C1-C8 alkoxy, m⁹ is 0 to 3, and m¹⁰ is 1 to 3; and (b) at least one alpha olefin copolymer. Said compositions have an improved photostability. Preferably said compositons have a photostability decrease of less than about 1/3 the photostability decrease of a similar composition that is the same as the sunscreen composition but not containing said alpha olefin copolymer.

The invention also relates to a sunscreen composition comprising:
(a) at least one dibenzoylmethane derivative UV-A absorbing agent, in particular of the formula: 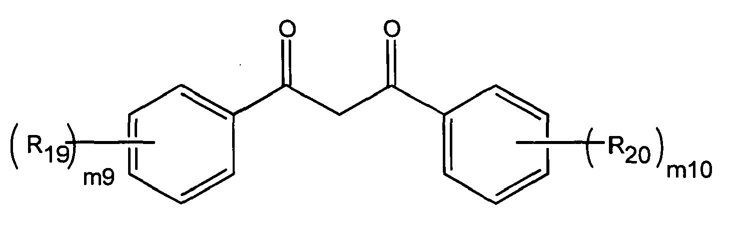 wherein R₁₉ and R₂₀, independently, are C1-C8 alkyl or C1-C8 alkoxy, m⁹ is 0 to 3, and m¹⁰ is 1 to 3; (b) at least one alpha olefin copolymer; and (c) at least one benzone derivative; wherein said composition preferably has a photostability decrease of less than about 1/3 the photostability decrease of a similar composition that is the same as the sunscreen composition but not containing said alpha olefin copolymer.

Finally, the invention also provides a method of increasing the photostability of a sunscreen composition comprising at least one dibenzoylmethane derivative UV-A absorbing agent, in particular of the formula: 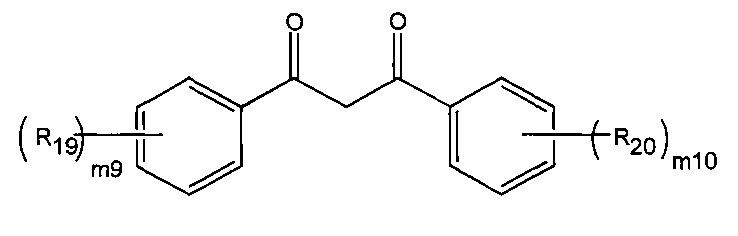 wherein R₁₉ and R₂₀, independently, are C1-C8 alkyl or C1-C8 alkoxy, m⁹ is 0 to 3, and m¹⁰ is 1 to 3; comprising adding thereto a photostabilizing effective amount of at least one alpha olefin copolymer.

### DETAILED DESCRIPTION OF THE INVENTION

The sunscreen composition comprises at least one dibenzoylmethane derivative UV-A absorbing agent. This is a compound comprising a dibenzoylmethane group and capable of absorbing radiation in the UV-A range (e.g., from about 320 to 400 nm). Examples of dibenzoylmethane derivative UV-A absorbing agents include those of the following formula: 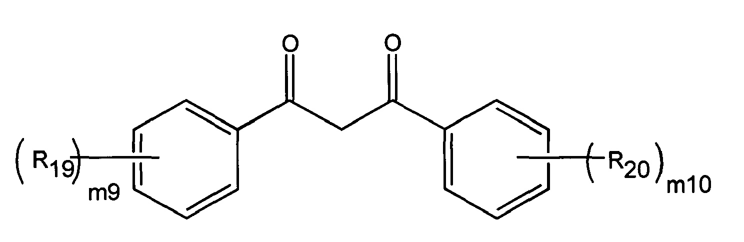 wherein R₁₉ and R₂₀, independently, are C1-C8 alkyl or C1-C8 alkoxy, m⁹ is 0 to 3, and m¹⁰ is 1 to 3. Examples of such compounds and their synthesis are disclosed in U.S. Patent No. 4,489,057 and include, but are not limited to, 4-(1,1-dimethylethyl)-4'-methoxydibenzoylmethane (avobenzone, PARSOL 1789), 2- 2-methyldibenzoylmethane, 4- methyl-dibenzoylmethane, 4-isopropyldibenzoylmethane, 4-tert-butyldibenzoylmethane, 4-tert-butyl-4'-methoxydibenzoylmethane, 2,4-dimethylbenzoylmethane, 2,5- dimethylbenzoylmethane, 4,4'-diisopropylbenzoylmethane, 2-methyl-5-isopropyl-4'-methoxydibenzoylmethane, 2-methyl-5-tert-butyl-4'-methoxydibenzoylmethane, 2,4-dimethyl-4'-methoxydibenzoylmethane, and 2,6-dimethyl-4-tert-butyl-4'-methoxydibenzoylmethane.

Preferably the dibenzoylmethane derivative UV-A absorbing agent is avobenzone. Also mixtures of the aforementioned compounds can be used.

In one embodiment, the amount of dibenzoylmethane derivative UV-A absorbing agent can range from about 0.1 % to about 20%, by weight of the composition (e.g., from about 1% to about 10%, by weight of the composition).

The composition also comprises an alpha olefin copolymer for photostabilization. In one embodiment, the alpha olefin copolymer photostabilizer is an alpha olefin/maleic anhydride copolymer, an alpha olefin/maleate copolymer, e.g. an alpha olefin/isopropyl maleate copolymer, or an alpha olefin/maleate/maleic anhydride copolymer used for photostabilization, e.g. an alpha olefin/isopropyl maleate/maleic anhydride copolymer. Preferably, the alpha olefin copolymer used for photostabilization is a C30-38 alpha olefin/isopropyl maleate/malcic anhydride copolymer, such as PERFORMA V1608 commercially available from New Phase Technologies.

The alpha olefin copolymer is used in the composition in a "photostabilizing effective amount," which means an amount sufficient to achieve a photostabilizing effect in the composition compared to compositions which are otherwise identical but do not contain said alpha olefin copolymer. Such amount will vary with the nature and amounts of the UV absorbing agents in the composition, as well as the other ingredients present. In general, said amount of alpha olefin copolymer may range up to about 20 % by weight of the composition, e.g., up to about 10%, up to 4% or up to about 2 % by weight of the composition.

In one embodiment, the composition further comprises one or more additional UV-A and/or UV-B absorbing agents. Examples of such agents include, but are not limited to methoxycinnamate derivatives such as octyl methoxycinnamate and isoamyl methoxycinnamate (however, compositions containing a combination of avobenzone and octyl methoxycinnamate have been found to be more difficult to photostabilize according to the invention); camphor derivatives such as 4-methyl benzylidene camphor, camphor benzalkonium methosulfate, and terephthalylidene dicamphor sulfonic acid; salicylate derivatives such as octyl salicylate, trolamine salicylate, and homosalate; sulfonic acid derivatives such as phenylbenzimidazole sulfonic acid; benzone derivatives such as dioxybenzone, sulisobenzone, and oxybenzone; benzoic acid derivatives such as aminobenzoic acid and octyldimethyl para-amino benzoic acid; octocrylene and other β,β-diphenylacrylates; dioctyl butamido triazone; titanium dioxide, zinc oxide; iron oxides; octyl triazone; butyl methoxydibenzoyl methane; drometrizole trisiloxane; triazoles, hydroxy benzophenones, and menthyl anthranilate. Other UV absorbers/reflectors useful herein can be found in Sagarin, Cosmetics Science and Technology, Chapter VIII, page 189 and the ICI Handbook, page 1672. A list of such compounds is also disclosed in U.S. Patent Number 4,919,934.

In one embodiment, the composition also comprises at least one UV-B absorbing agent. In particular, the composition may comprise a benzone derivative such as dioxybenzone, sulisobenzone, or oxybenzone, preferably oxybenzone.

In a further embodiment, the composition additionally comprises octocrylene or another β,β-diphenylacrylate, preferably octocrylene.

In another embodiment, the composition also comprises at least one triazine. U.S. Patent No. 5,955,060 discloses triazine compounds and their preparation. In particular, the triazine is 2,4-bis{[4-(2-ethyl-hexyloxy)-2-hydroxy]-phenyl}-6-(4-methoxyphenyl)-(1,3,5)-triazine, sold commercially as TINOSORB S by Ciba Specialty Chemicals Corporation, Greensboro, North Carolina, USA.

Such additional UV absorbing agents may each be present in the composition in the general range of about 0.1 % to about 30% by weight of the composition (e.g., from about 1% to about 20% by weight of the composition). The total concentration of all such agents should be based on the desired sunscreen protection factor ("SPF") level, as well known in the art.

The composition may further comprise one or more other cosmetically active agent(s), as known in the art. A "cosmetically active agent" is a compound that has a cosmetic or therapeutic effect on the skin, e.g., agents to treat wrinkles, acne, or to lighten the skin. In one embodiment, the agent is selected from, but not limited to, the group consisting of: hydroxy acids; benzoyl peroxide; sulfur resorcinol; D-panthenol; hydroquinone; anti-inflammatory agents; skin lightening agents; antimicrobial and antifungal agents such a miconazole, ketoconazole, and elubial; vitamins such as ascorbic acid; tocopherols and tocotrienols such as tocopheryl acetate; retinoids such retinol, retinal, retinyl palmitate, retinyl acetate, and retinoic acid; hormones such as estrogens and dihydroxyandrostene dione; 2-dimethylaminoethanol; lipoic acid; amino acids such a proline and tyrosine; lactobionic acid; self-tanning agents such as dihydroxy acetone; dimethyl aminoethanol; acetyl-coenzyme A; niacin; riboflavin; thiamin; ribose; electron transporters such as NADH and FADH2; botanical extracts such as ginkgo biloba, aloe vera, and soy; and derivatives thereof. The cosmetically active agent may be present in an amount of about 0.001% to about 20% by weight of the composition, e.g., about 0.01% to about 10%, such as about 0.1% to about 5% by weight of the composition.

Examples of hydroxy acids include, but are not limited, to (i) alpha-hydroxy acids such as glycolic acid, lactic acid, malic acid, citric acid, and tartaric acid, (ii) betahydroxy acids such as salicylic acid, and/or (iii) polyhydroxy acids. See, e.g., EP 273,202.

Examples of derivatives of ascorbic acid include, but are not limited to, ascorbyl palmitate, magnesium ascorbyl phosphate, sodium ascorbyl phosphate, zinc ascorbyl phosphate, ascorbyl glucoside, sodium ascorbate, and ascorbyl polypeptide. An example of a derivative of hydroquinone is arbutin.

The composition may also comprise one or more of the following: antioxidants (e.g., ascorbic acid, tocopherols, polyphenols, tocotrienols, BHA, and BHT), chelating agents (e.g., EDTA), and preservatives (e.g., parabens). Examples of suitable antioxidants, preservatives, and chelating agents are listed in pp. 1612-13, 1626, and 1654-55 of the ICI Handbook*.* In addition, the composition may contain conventional cosmetic adjuvants, such as dyes, opacifiers (e.g., titanium dioxide), pigments, and fragrances.

The composition may optionally comprise additional photostabilizers, such as a diester or polyester of a naphthalene dicarboxylic acid. These are not required, however. Examples of diesters and polyesters of a naphthalene dicarboxylic acid are compounds of formulae (X) or (XI): 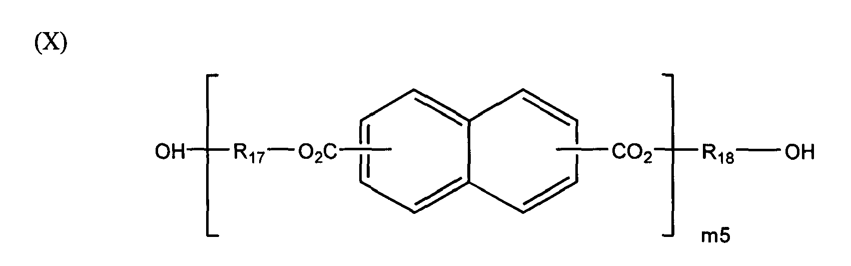 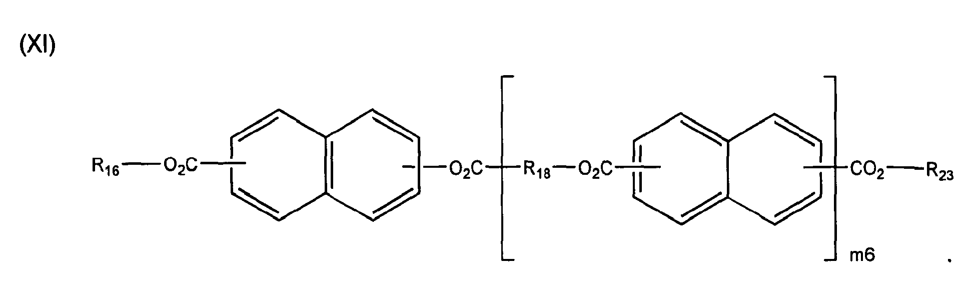 wherein R₁₆ and R₂₃, independently, are selected from the group consisting of a C1-C22 alkyl, a diol having the structure HO-R₁₈-OH, and a polyglycol having the structure HO-R₁₇-(-O-R₁₈-)m5-OH; R₁₇ and R₁₈, independently, are C1-C6 alkenyl; and m⁵ and m⁶, independently, are each in the range of 1 to about 100. Examples, including the synthesis, of such diesters or polyesters of naphthalene dicarboxylic acid are described in U.S. Patent No. 5,993,789, and include, but not limited to, diethylhexyl naphthalate (commercially available as HALLBRITE TQ from C.P. Hall Company, Bedford Park, Illinois, USA). See Bonda, et al., Allured's Cosmetic & Toiletries Magazine, 115(6):37-45 (2000) disclosing the uses of such compounds in sunscreen compositions. In one embodiment, the diester or polyester of a naphthalene dicarboxylic acid can range from about 0.1% to about 30%, by weight, of the total composition (e.g., from about 1% to about 10%, by weight).

The composition may be used by topically administering to a mammal, e.g., by the direct laying on or spreading of the composition on the skin or hair of a human. The composition may comprise a cosmetically-acceptable topical carrier. The term "cosmetically-acceptable topical carrier" refers to a carrier for topical use that is capable of having the other ingredients dispersed or dissolved therein, and possessing acceptable safety properties.

The composition may be used for a variety of cosmetic purposes, including, but not limited to, protection the skin or hair from UV radiation. The composition may be made into a wide variety of product types. These include, but are not limited to lotions, creams, gels, sticks, sprays, ointments, mousses, and cosmetics/make-up. Such products may comprise several types of carrier systems including, but not limited to single phase solutions (e.g., oil based solutions), emulsions, and gels.

The composition may be prepared using methodology well known in the art.

According to the invention, the sunscreen composition is photostabilized by the presence of the alpha olefin copolymer, whether or not additional photostabilizers are present in the composition. Generally, the composition of the invention has a photostability decrease of less than about 10 %, preferably less than about 5%, more preferably less than about 2%. Photostability decrease can be measured as follows.

The photostability is regularly assessed by determining the loss of efficacy (in percentage) of the tested product, that is, (A): loss of efficacy (%) = [(UV protection before radiation - UV protection after irradiation)/UV protection before radiation] x 100. In the case of a photo-unstable formulation transmission increases during radiation, and therefore, a decrease of the UV protection is observed. The measuring technique usually consists in determining the monochromatic protection factors at 5 nm wavelength intervals throughout the UVA and/or the UVB regions of the electromagnetic spectrum. This can be accomplished by using a light source which emits a continuous spectrum in the UV, a monochromator to select individual measurement wavelengths and a UV detector to measure UV light intensities at each wavelength increment.

The photostability decrease of the composition when alpha olefin copolymers are used for photostabilization preferably is less than about 1/3, for example less than about 1/5, preferably less than about 1/10, of the photostability decrease of a similar composition containing all of the same ingredients in all of the same amounts but not containing the alpha olefin copolymer. That is, said similar composition is the same in all respects except for the absence of alpha olefin copolymer.

### EXAMPLES

The following compositions according to the invention (Examples 1-8) and comparative compositions (Examples A-C) were prepared.

The photostability, that is the amount of loss or decrease in photostability of the products according to the examples was determined after 10 MED (minimal erythemal dose) irradiation by using the above equation (A). Each product was tested three times. 0.75 µl/cm² of the compositions were placed on a roughened glass plate as for example available from THUET BIECHELIN LABS, Glodelsheim France (70 mm x 70 mm x 1 mm). These glass plates have a textured upper surface similar to that of the human skin so that the compositions can be distributed thereon in a similar manner. As a UV light source a Xenon lamp (400 W solar simulator ORIEL) filtered with a SCHOTT filter WG 320 1 mm and a MTO filter was used. The samples were irradiated at 10 x 25 mJ/cm². For the photostability, that is degradation measurement of the UV filter in the sunscreen composition the light source complied with the C.O.L.I.P.A. SPF test method definition.

To measure transmission a UV/visible spectroradiometer model 752 (Optronic Laboratory) comprising an external integrating sphere, a double monochromator and a photomultiplier were used. The command system was controlled by a microprocessor, and the lighting system was provided by an unfiltered 75 watt OSRAM Xenon lamp.

For the transmission measurement light sources can be used which emit continuous spectral irradiance between 290 and 400 nm with the sufficient intensity at all wavelengths to produce a signal that can be measured accurately by the detector (within a 2 to 3 absorbance units range). A monochromator used for this purpose should be able to scan all wavelengths from 290 to 400 nm and should contain optical components as designed for use with UV light. The bandwidth should be 5 nm or less.

On the quartz plate the samples were left for half an hour in the dark at room temperature to ensure self-levelling before measuring transmission each 5 nm. Then SBF and UVA protection were determined before and after irradiation.

**TABLE 1**

| **INGREDIENT** | **Example 1** | **Example 2** |
|---|---|---|
| WATER | 45.620 | 47.970 |
| Sclorotium Gum | 0.400 | 0.400 |
| DIPOTASSIUM GLYCYRRHETINATE | 0.050 | 0.050 |
| Butylene Glycol | 3.000 | 3.000 |
| Glycerine | 4.000 | 4.000 |
| DISODIUM EDTA | 0.200 | 0.200 |
| Caprylyl Glycol | 0.500 | 0.500 |
| HOMOSALATE | 8.000 | 8.000 |
| ETHYLHEXYL SALICYLATE | 4.000 | 4.000 |
| AVOBENZONE | 3.000 | 3.000 |
| BENZOPHENONE -3 | 6.000 | 6.000 |
| OCTOCRYLENE | 3.000 | 3.000 |
| DIETHYLHEXYL 2,6-NAPHTALATE | 2.350 | 0.000 |
| Adipic acid/Diethylene Glycol/Glycerine Crosspolymer | 3.000 | 3.000 |
| PVP-HEXADECENE COPOLYMER | 3.000 | 3.000 |
| DIMETHICONE & TRIMETHYLSILOXYSILICATE | 1.000 | 1.000 |
| C30-38 Olefin/isopropyl Maleate/MA copolymer | 2.000 | 2.000 |
| GLYCERYL STEARATE & PEG-100 STEARATE | 1.000 | 1.000 |
| Potassium Cetyl Phosphate | 0.500 | 0.500 |
| Behenyl Alcohol | 0.400 | 0.400 |
| TOCOPHERYL ACETATE | 0.500 | 0.500 |
| Bisabolol | 0.500 | 0.500 |
| DIMETHICONE, 200 fluid, 50 cst. | 2.000 | 2.000 |
| Aqua/Polysorbate 60/Squalane/Sodium Acryloyldimethyltaurate Hydroxyethylacrylates Copolymer | 1.300 | 1.300 |
| Silica | 3.000 | 3.000 |
| Dimethicone Trisiloxane | 1.000 | 1.000 |
| Methylisothiazolinone | 0.100 | 0.100 |
| Amanduline | 0.100 | 0.100 |
| Perfum | 0.400 | 0.400 |
| Sodium Hydroxide | 0.080 | 0.080 |
| | 100 | 100.000 |
| PHOTOSTABILITY | OK | OK |
| PHOTOSTABILITY DECREASE | -2.90% | -2.90% |

**TABLE 2**

| **INGREDIENT** | **EXAMPLE A** | **EXAMPLE 3** | **EXAMPLE B** | **EXAMPLE 4** | **EXAMPLE 5** |
|---|---|---|---|---|---|
| WATER | 58.45 | 57.95 | 45.6 | 44.9 | 45.5 |
| Sclorotium Gum | 0.4 | 0.4 | 0.4 | 0.4 | 0.4 |
| DIPOTASSIUM GLYCYRRHETINATE | 0.05 | 0.05 | 0.05 | 0.05 | 0.05 |
| Butylene Glycol | 3.000 | 3.000 | 3.000 | 3.000 | 3.000 |
| Glycerine | 4.000 | 4.000 | 4.000 | 4.000 | 4.000 |
| DISODIUM EDTA | 0.200 | 0.200 | 0.200 | 0.200 | 0.200 |
| Caprylyl Glycol | 0.500 | 0.500 | 0.500 | 0.500 | 0.500 |
| HOMOSALATE | 6.000 | 6.000 | 8.000 | 8.000 | 8.000 |
| ETHYLHEXYL SALICYLATE | 0 | 0 | 4.000 | 4.000 | 4.000 |
| AVOBENZONE | 2.000 | 2.000 | 3.000 | 3.000 | 3.000 |
| BENZOPHENONE -3 | 2.000 | 2.000 | 6.000 | 6.000 | 6.000 |
| OCTOCRYLENE | 2.500 | 2.500 | 3.000 | 3.000 | 3.000 |
| NAPHTALATE | 2.000 | 2.000 | 2.350 | 2.350 | 2.350 |
| Adipic acid/Diethylene Glycol/Glycerine Crosspolymer | 2.500 | 2.500 | 3.000 | 3.000 | 3.000 |
| PVP-HEXADECENE COPOLYMER | 2.500 | 2.500 | 3.000 | 3.000 | 3.000 |
| DIMETHICONE & TRIMETHYLSILOXYSILI CATE | 1.000 | 1.000 | 1.000 | 1.000 | 1.000 |
| C30-38 Olefin/Isopropyl Maleate/MA Polymer | 0 | 2.000 | 0 | 2.000 | 2.000 |
| GLYCERYL STEARATE & PEG-100 STEARATE | 1.500 | 0 | 1.500 | 0 | 1.000 |
| Potassium Cetyl Phosphate | 0.500 | 0.500 | 1.000 | 1.200 | 0.500 |
| BEHENYL ALCOHOL | 0.400 | 0.400 | 0.400 | 0.400 | 0.400 |
| TOCOPHERYL ACETATE | 0.500 | 0.500 | 0.500 | 0.500 | 0.500 |
| BISABOLOL | 0.500 | 0.500 | 0.500 | 0.500 | 0.500 |
| DIMETHICONE, 200 fluid, 50 cst. | 2.000 | 2.000 | 2.000 | 2.000 | 2.000 |
| CETHYL DIMETHICONE | 1.000 | 1.000 | 1.000 | 1.000 | 0 |
| Aqua/Polysorbate 60/Squalane/Sodium Acryloyldimethyltaurate Hydroxyethylacrylates Copolymer | 2 | 2 | 1.5 | 1.5 | 1.5 |
| SILICA | 3.000 | 3.000 | 3.000 | 3.000 | 3.000 |
| Dimethicone Trisiloxane | 1.000 | 1.000 | 1.000 | 1.000 | 1.000 |
| METHYLISOTHIAZOLIN ONE | 0.1 | 0.1 | 0.1 | 0.1 | 0.1 |
| Amanduline | 0.1 | 0.1 | 0.1 | 0.1 | 0.1 |
| Perfum | 0.300 | 0.300 | 0.300 | 0.300 | 0.400 |
| | | | | | |
| PHOTOSTABILITY | NOT OK | OK | NOT OK | OK | OK |
| PHOTOSTABILITY DECREASE | -32.10% | -2.60% | -17.20% | -6.40% | -2.90% |

**TABLE 3**

| **INGREDIENT** | **EXAMPLE C** | **EXAMPLE 6** | **EXAMPLE 7** | **EXAMPLE 8** |
|---|---|---|---|---|
| Aqua | 55.6 | 56.6 | 54.6 | 55.1 |
| Xanthan Gum | 0.150 | 0.150 | 0.150 | 0.150 |
| Butylene glycol | 3.000 | 3.000 | 3.000 | 3.000 |
| Glycerin | 4.00 | 4.00 | 4.00 | 4.00 |
| Scleroticum Gum | 0.40 | 0.40 | 0.40 | 0.40 |
| Sodium Polyacrylate | 0.30 | 0.30 | 0.30 | 0.30 |
| Disodium EDTA | 0.100 | 0.100 | 0.100 | 0.100 |
| Styrene/Acrylates copolymer | 1.000 | 1.000 | 1.000 | 1.000 |
| Caprylyl glycol | 0.500 | 0.500 | 0.500 | 0.500 |
| Cetyl alcohol, Glyceryl Stearate, Steareth-20, Ceteth-20, PEG-75 Stearate | 3.000 | 0.000 | 3.000 | 3.000 |
| C30-38 olefin/Isopropyl Maleate/MA copolymer | 0.000 | 2.000 | 1.000 | 0.500 |
| Potassium cetyl phosphate | 0.500 | 0.500 | 0.500 | 0.500 |
| cetearyl alcohol | 0.250 | 0.250 | 0.250 | 0.250 |
| Dicaprylyl Carbonate | 1.500 | 1.500 | 1.500 | 1.500 |
| Butyrospermum parkii | 0.500 | 0.500 | 0.500 | 0.500 |
| Stearyl dimethicone | 0.800 | 0.800 | 0.800 | 0.800 |
| PVP eicosene copolymer | 3.000 | 3.000 | 3.000 | 3.000 |
| Homosalate | 8.000 | 8.000 | 8.000 | 8.000 |
| Tocopherol acetate | 0.500 | 0.500 | 0.500 | 0.500 |
| Benzophenone-3 | 5.000 | 5.000 | 5.000 | 5.000 |
| BMDBM | 3.000 | 3.000 | 3.000 | 3.000 |
| Octocrylene | 3.000 | 3.000 | 3.000 | 3.000 |
| Bis-ethyl hexylphenol methoxyphenyl triazine | 2.500 | 2.500 | 2.500 | 2.500 |
| Cyclopentasiloxane, Cyclohexasiloxane | 1.500 | 1.500 | 1.500 | 1.500 |
| Diethylhexyl 2,6-Naphthalate | 0.500 | 0.500 | 0.500 | 0.500 |
| Methylisothiazolinone | 0.100 | 0.100 | 0.100 | 0.100 |
| Parfum | 0.300 | 0.300 | 0.300 | 0.300 |
| Silica | 1.000 | 1.000 | 1.000 | 1.000 |
| | 100.00 | 100.00 | 100.00 | 100.00 |
| PHOTOSTABILITY | NOT OK | OK | OK | OK |
| PHOTOSTABILITY DECREASE | -15.40% | -2.70% | 1.50% | 4.60% |

For each composition, photostability decrease was measured as described above. The compositions of Examples 1-8 had acceptable photostability, losing at most 6.40% of their photostability. In contrast, the photostabilities of the compositions of comparative Examples A-C decreased by at least 15.40 %. The composition of Example A had a photostability decrease of 32.10%. All of the comparative examples contained diethylhexyl 2,6-naphthalate.

## Claims

**1.** Use of (b) at least one alpha olefin copolymer as a photostabilizing agent in a sunscreen composition comprising (a) at least one dibenzoylmethane derivative UV-A absorbing agent.

**2.** The use of claim 1 wherein said dibenzoylmethane derivative UV-A absorbing agent has the formula: 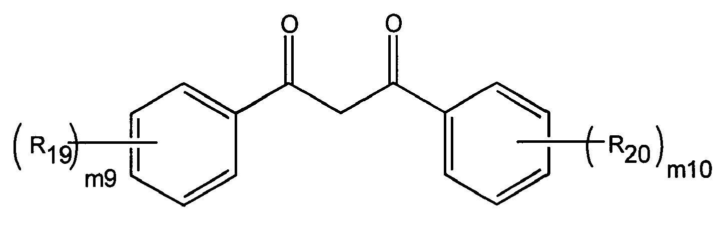 wherein R₁₉ and R₂₀, independently, are C1-C8 alkyl or C1-C8 alkoxy, m⁹ is 0 to 3, and m¹⁰ is 1 to 3.

**3.** The use of claim 1 or 2, wherein said dibenzoylmethane derivative UV-A absorbing agent is avobenzone.

**4.** The use according to any of the preceding claims, wherein said alpha olefin copolymer is an alpha olefin/maleic anhydride copolymer.

**5.** The use according to any of the preceding claims, wherein said alpha olefin copolymer is a C30-38 alpha olefin/isopropyl maleate/maleic anhydride copolymer.

**6.** The use according to any of the preceding claims, wherein said sunscreen composition further comprises at least one UV-B filter.

**7.** The use according to any of the preceding claims, wherein said sunscreen composition further comprises octocrylene.

**8.** The use according to any of the preceding claims, wherein said sunscreen composition further comprises at least one triazine derivative.

**9.** The use of claim 8, wherein said triazine derivative is 2,4-bis{[4-(2-ethylhexyloxy)-2-hydroxy]-phenyl}-6-(4-methoxyphenyl)-(1,3,5)-triazine.

**10.** The use according to any of the preceding claims, wherein said sunscreen composition further comprises:
(c) at least one benzone derivative.

**11.** The use of claim 10 wherein said benzone derivative is selected from the group consisting of dioxybenzone, sulisobenzone, and oxybenzone.

**12.** The use according to any of the preceding claims wherein said composition has a photostability decrease of less than about 1/3 of the photostability decrease of a composition that is the same as the sunscreen composition but not containing said alpha olefin copolymer.

**13.** A sunscreen composition comprising:
(a) a dibenzoylmethane derivative UV-A absorbing agent, e.g. of the formula: 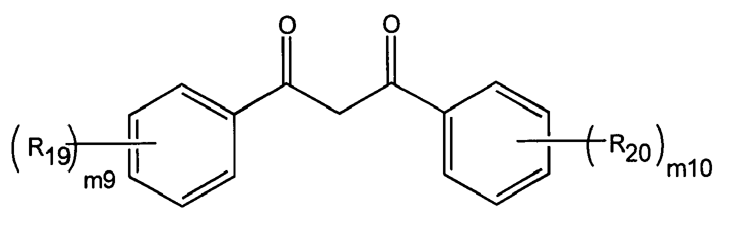 wherein R₁₉ and R₂₀, independently, are C1-C8 alkyl or C1-C8 alkoxy, m⁹ is 0 to 3, and m¹⁰ is 1 to 3; and
(b) an alpha olefin copolymer;
wherein said composition has a photostability decrease of less than about 1/3 the photostability decrease of a similar composition that is the same as the sunscreen composition but not containing said alpha olefin copolymer.

**14.** The composition of claim 13, wherein said dibenzoylmethane derivative UV-A absorbing agent is avobenzone.

**15.** The composition of claim 13, wherein said alpha olefin copolymer is an alpha olefin/maleic anhydride copolymer.

**16.** The composition of claim 13, wherein said alpha olefin copolymer is a C30-38 alpha olefin/isopropyl maleate/maleic anhydride copolymer.

**17.** The composition of claim 13 further comprising a UV-B filter.

**18.** The composition of claim 13 further comprising octocrylene.

**19.** The composition of claim 13 further comprising a triazine derivative.

**20.** The composition of claim 19, wherein said triazine derivative is 2,4-bis{[4-(2-ethyl-hexyloxy)-2-hydroxy]-phenyl}-6-(4-methoxyphenyl)-(1,3,5)-triazine.

**21.** A sunscreen composition comprising:
(a) a dibenzoylmethane derivative UV-A absorbing agent, e.g. of the formula: 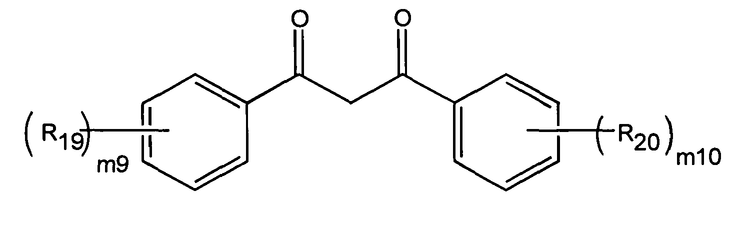 wherein R₁₉ and R₂₀, independently, are C1-C8 alkyl or C1-C8 alkoxy, m⁹ is 0 to 3, and m¹⁰ is 1 to 3;
(b) an alpha olefin copolymer; and
(c) a benzone derivative;
wherein said composition has a photostability decrease of less than about 1/3 the photostability decrease of a similar composition that is the same as the sunscreen composition but not containing said alpha olefin copolymer.

**22.** The composition of claim 21, wherein said dibenzoylmethane derivative UV-A absorbing agent is avobenzone.

**23.** The composition of claim 21, wherein said alpha olefin copolymer is an alpha olefin/maleic anhydride copolymer.

**24.** The composition of claim 21, wherein said alpha olefin copolymer is a C30-38 alpha olefin/isopropyl maleate/maleic anhydride copolymer.

**24.** The composition of claim 21 further comprising octocrylene.

**25.** The composition of claim 21 further comprising a triazine derivative.

**26.** The composition of claim 25, wherein said triazine derivative is 2,4-bis{[4-(2-ethyl-hexyloxy)-2-hydroxy]-phenyl}-6-(4-methoxyphenyl)-(1,3,5)-triazine.

**27.** The composition of claim 21 wherein said benzone derivative is selected from the group consisting of dioxybenzone, sulisobenzone, and oxybenzone.

**28.** A method of increasing the photostability of a sunscreen composition comprising a dibenzoylmethane derivative UV-A absorbing agent, e.g. of the formula: 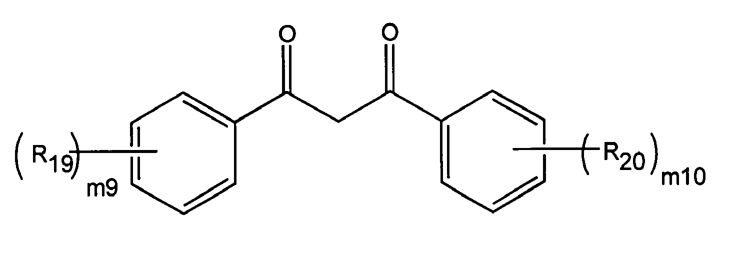 wherein R₁₉ and R₂₀, independently, are C1-C8 alkyl or C1-C8 alkoxy, m⁹ is 0 to 3, and m¹⁰ is 1 to 3; comprising adding thereto a photostabilizing effective amount of an alpha olefin copolymer.

**29.** The method of claim 28, wherein said dibenzoylmethane derivative UV-A absorbing agent is avobenzone.

**30.** The method of claim 28, wherein said alpha olefin copolymer is an alpha olefin/mateic anhydride copolymer.

**31.** The method of claim 28, wherein said alpha olefin copolymer is a C30-38 alpha olefin/isopropyl maleate/maleic anhydride copolymer.

**32.** The method of claim 28 further comprising octocrylene.

**33.** The method of claim 28 further comprising a triazine derivative.

**34.** The method of claim 33, wherein said triazine derivative is 2,4-bis{[4-(2-ethylhexyloxy)-2-hydroxy]-phenyl}-6-(4-methoxyphenyl)-(1,3,5)-triazine.

**35.** The method of claim 28 wherein said composition further comprises a benzone derivative selected from the group consisting of dioxybenzone, sulisobenzone, and oxybenzone.

**36.** The method of claim 28, wherein said photostabilizing effective amount is up to about 20 % by weight of the composition.
